# DEMANDE DE BREVET EUROPEEN

(11) **EP 1 348 418 A1**
(43) Date de publication de la demande: **01.10.2003**
(21) Numéro de dépôt: 03290561.4
(22) Date de dépôt: 07.03.2003
(51) Int. Cl.: A61K 7/06, A61K 7/42, A61K 7/48

(54) **Composition cosmétique ou dermatologique contenant l'association d'un tetrahydrocurcuminoide et d'une huile amidée**

(30) Priorité: 28.03.2002 FR 0203932
(71) Demandeur: L'OREAL, 75008 Paris (FR)
(72) Inventeur: Seyler, Nathalie, 94700 Maisons-Alfort (FR); Fiandino, Cécile, 75013 Paris (FR); Candau, Didier, 91570 Bievres (FR)
(74) Mandataire: Miszputen, Laurent

(57) **Abrégé**

L'invention concerne une composition cosmétique ou dermatologique contenant un support comprenant au moins une phase grasse caractérisée par le fait qu'elle contient au moins un dérivé ou un mélange de dérivés de la 1,7-bisphényl heptane-3,5-dione de structure particulière et au moins une huile présentant dans sa structure au moins un motif amide

L'invention concerne également ses utilisations en cosmétique et dermatologie notamment pour prévenir et/ou lutter contre les effets néfastes des UV et de la pollution sur les matières kératiniques humaines et notamment pour prévenir et/ou traiter le photovieillissement de la peau.

L'invention se rapporte également à un procédé de solubilisation d'un dérivé ou d'un mélange de dérivés de la 1,7-bisphényl heptane-3,5-dione de structure particulière par au moins une huile présentant dans sa structure au moins un motif amide.

## Description

L'invention concerne une composition cosmétique ou dermatologique contenant un support comprenant au moins une phase grasse caractérisée par le fait qu'elle contient au moins un dérivé ou un mélange de dérivés de la 1,7-bisphényl heptane-3,5-dione de structure particulière et au moins une huile présentant dans sa structure au moins un motif amide ainsi que ses utilisations en cosmétique et dermatologie notamment contre les effets néfastes des UV et de la pollution sur les matières kératiniques humaines et plus particulièrement pour prévenir et/ou traiter le photovieillissement de la peau.

L'invention se rapporte également à un procédé de solubilisation d'un dérivé ou d'un mélange de dérivés de la 1,7-bisphényl heptane-3,5-dione de structure particulière par au moins une huile présentant dans sa structure au moins un motif amide.

Au cours du temps, il apparaît différents signes sur la peau, très caractéristiques du vieillissement, se traduisant notamment par une modification de la structure et des fonctions cutanées.

Ce vieillissement, de nature physiologique, peut-être accéléré par des facteurs environnementaux tels qu'une exposition répétée de la peau à la lumière solaire, et notamment aux rayonnements ultraviolets A, à la pollution, en particulier ou aux particules de diesel ou à la fumée de cigarette. L'action de l'environnement sur les constituants de la peau (fibres, cellules, enzymes) et sur le sébum sécrété par la peau entraîne en particulier la formation de radicaux libres oxygénés. Or, ces radicaux provoquent des dégâts oxydatifs importants, notamment dans les membranes cellulaires (perméabilité des membranes), les noyaux des cellules (destruction de l'ADN), et les tissus, en particulier le tissu conjonctif (dégradation des fibres d'élastine et de collagène). Ces dégâts conduisent notamment à une perte de fermeté et d'élasticité de la peau.

Il a en outre été suggéré que les radicaux libres pouvaient intervenir dans le processus de fabrication de la mélanine conduisant à la pigmentation de la peau.

Le mécanisme de formation de la mélanine est particulièrement complexe et fait intervenir schématiquement les principales étapes suivantes :
Tyrosine ---> Dopa ---> Dopaquinone ---> Dopachrome ---> Mélanine

La tyrosinase (monophénol dihydroxyl phénylalanine : oxygen oxydo-reductase EC 1.14.18.1) est l'enzyme essentielle intervenant dans cette suite de réactions. Elle catalyse notamment la réaction de transformation de la tyrosine en Dopa (dihydroxyphénylalanine) grâce à son activité hydroxylase. Or, certains auteurs pensent que l'étape d'hydroxylation de la tyrosine en Dopa pourrait être initiée par des radicaux OH° (C. Montastier et al., Méthodes d'objectivation des effets des agents dépigmentants chez l'homme, J. Med. Esth. et Chir. Derm., Vol. XXII, 86, Juin 1995, pp. 93-103).

Les radicaux libres formés sous l'effet de facteurs environnementaux pourraient donc entraîner une augmentation de la formation de la mélanine, et provoquer ou accentuer ainsi certaines hyperpigmentations indésirables telles que les mélasmas idiopathiques, survenant lors de la grossesse ("masque de grossesse" ou chloasma) ou d'une contraception oestro-progestative ou encore les hyperpigmentations localisées, telles que les taches pigmentaires séniles dites lentigo actiniques.

Il est donc nécessaire de protéger la peau contre ces radicaux libres.

Cette fonction protectrice est normalement assurée par des enzymes présentes dans le tissu cutané. Toutefois, dans certaines circonstances, le rôle de ces enzymes n'est pas suffisant pour bloquer totalement l'action destructrice des radicaux libres.

Les tétrahydrocurcuminoides obtenus par réduction de la curcumine ou synthétisés tels que décrits dans les articles « Synthesis and antibacterial activity of tetrahydrocurcuminoids ; S Venkateswarlu, M Rambabu, GV Subbaraju and S Satyanarayana ; Asian Journal of Chemistry-2000-1-141-144.» , « Synthesis of naturally occuring curcuminoids and related compounds ; U Pedersen, PB Rasmussen, SO Lawesson ; Liebigs Ann. Chem.-1985-1557-1569., les demandes de brevet JP02051595, JP02069431, JP02049747 et JP02128133, le brevet US 5,266,344 et la demande WO00/61162 ont déjà été utilisés en cosmétique comme agent protecteur de la peau contre les effets des radiations UV (JP06128133), comme agent antioxydant et/ou comme agent anti-radicaux libres dans la demande de brevet WO97/03674. Ils sont également utilisés dans la demande WO99/55352 dans des compositions blanchissantes ou dépigmentantes de la peau en association avec un dépigmentant.

On connaît également de la demande de brevet EP1108419 des compositions cosmétiques pour application topique sur la peau contenant au moins un tétrahydrocurcuminoide comme la tétrahydrocurcumine et/ou la tétrahydrodéméthoxycurcumine et/ou la tétrahydro bis-déméthoxycurcumine ou leurs mélanges. Elles sont utilisées pour protéger les matières kératiniques humaines contre les effets néfastes dus à divers facteurs de l'environnement : radiations UV, fumées, ozone, substances polluantes. Elles sont également préconisées pour lutter contre la formation des radicaux libres et le vieillissement de la peau intrinsèque ou extrinsèque. On sait également dans la demande WO99/22728 que les tétrahydrocurcuminoides sont également actifs comme inhibiteurs de 5α-réductase.

Au cours de ses recherches, la demanderesse a constaté que certains dérivés de la 1,7-bisphényl heptane-3,5-dione dont les tétrahydrocurcuminoides actifs comme agents antioxydants et/ou comme agents anti-radicaux libres perdaient de façon sensible leur efficacité dans les supports cosmétiques courants comprenant au moins une phase grasse (notamment les émulsions) en raison de leur très faible solubilité dans la plupart des huiles couramment utilisées et conduisaient à des formulations microscopiquement instables dans le temps du fait qu'il cristallisent et se déposent dans le support. Cette instabilité des tétrahydrocurcuminoides peut en outre modifier l'aspect des compositions les contenant.

Parmi les huiles couramment utilisées en cosmétique, on peut citer les huiles minérales comme celles du type paraffine (MARCOL 82 de Esso) ou les hydrocarbures comme l'isohexadécane (PERMETHYL 101A de BAYER) ; les huiles d'origine végétale comme l'huile d'amande d'abricot raffinée (MP301 de Nestlé) ; les cires liquides comme la cire liquide de jojoba (Pure Golden Jojoba Oil de DESERT WHALE) ; les esters d'acides gras comme le maléate de dicaprylyle (BERNEL ESTER de DOM), le carbonate de dicaprylyle (CETIOL CC de Cognis), le trimellitate de tridécyle (DUB TMTD de Stéarine Dubois), le maléate de dioctyle (CERAPHYL CM5 de Rhodia Chimie), le palmitate d'isopropyle, l'isononanoate d'isononyle (WICKENOL 151 de Alzo) et les benzoates d'alcools en C₁₂-C₁₅ (FINSOLV TN de WITCO) ; les triglycérides d'acides gras en C₆-C₁₈ tels que les triglycérides d'acide caprylique/caprique (MYRITOL 318 de Cognis).

La Demanderesse a maintenant découvert, de façon inattendue et surprenante, que les hùiles présentant dans leur structure au moins une fonction amide permettaient de solubiliser dans des quantités importantes les dérivés de la 1,7-bis-phényl heptane-3,5-dione de formule (I), définie ci-après, tels que les tétrahydrocurcuminoides dans des supports cosmétiques comprenant une phase grasse comme les émulsions et d'obtenir des formulations stables dans le temps et présentant une meilleure efficacité à l'égard des matières kératiniques humaines contre les effets nefastes des UV et/ou de la pollution en particulier contre le photo-vieillissement de la peau.

Par matières kératiniques humaines, on entend la peau, les lèvres, le cuir chevelu, les cheveux, les cils et sourcils ainsi que les ongles.

Cette découverte est à la base de la présente invention.

Un premier objet de l'invention concerne une composition cosmétique ou dermatologique contenant un support comprenant au moins une phase grasse caractérisée par le fait qu'elle contient au moins un dérivé ou un mélange de dérivés de la 1,7-bisphényl heptane-3,5-dione de formule (I), définie ci-après et au moins une huile présentant dans sa structure au moins un motif amide.

Un autre objet de l'invention concerne l'utilisation cosmétique d'une composition selon l'invention pour prévenir ou lutter contre les effets néfastes des radiations UV et/ou de la pollution sur les matières kératiniques humaines et plus particulièrement la peau.

Par "pollution", on entend aussi bien la pollution "extérieure", due par exemple aux particules de diesel, à l'ozone ou aux métaux lourds, que la pollution "intérieure" qui peut notamment être due aux émissions de solvants de peinture, de colles de moquette, d'isolants ou de papiers peints (tels que toluène, styrène, xylène ou benzaldéhyde), ou bien à la fumée de cigarette. Tous ces polluants sont en effet susceptibles de générer, directement ou indirectement, des radicaux libres.

Un autre objet de l'invention concerne l'utilisation d'une composition pour la préparation d'un produit destiné à prévenir la formation des radicaux libres au contact de la peau et/ou à neutraliser les radicaux libres déjà formés.

Un autre objet de l'invention concerne l'utilisation d'une composition selon l'invention pour la préparation d'un produit destiné à traiter les peaux endommagées par les effets néfastes des radiations UV et de la pollution et notamment à traiter les peaux desséchées, craquelées, irritées ou brûlées par le soleil et/ou la pollution.

Un autre objet de l'invention concerne l'utilisation d'une composition selon l'invention pour la préparation d'un produit destiné à prévenir et/ou traiter les signes du vieillissement cutané et notamment destiné à prévenir et/ou traiter la perte de fermeté et/ou d'élasticité de la peau.

L'invention a aussi pour objet l'utilisation cosmétique de cette composition pour prévenir ou réduire la formation de taches pigmentaires et/ou pour blanchir ou dépigmenter la peau.

Un autre objet de l'invention concerne l'utilisation d'au moins une huile présentant dans sa structure au moins un motif amide dans une composition cosmétique ou dermatologique contenant au moins un dérivé ou un mélange de la 1,7-bisphényl heptane-3,5-dione de formule (I) pour solubiliser ledit dérivé ou un mélange de dérivés de formule (I).

L'invention concerne également un procédé de solubilisation d'au moins un dérivé ou un mélange de dérivés de la 1,7-bisphényl heptane-3,5-dione de formule (I) dans une composition cosmétique ou dermatologique comportant au moins une phase grasse consistant à utiliser au moins une huile présentant dans sa structure au moins un motif amide dans une quantité suffisante permettant de solubiliser à elle seule la totalité du ou des dérivés de la 1,7-bisphényl heptane-3,5-dione.

D'autres objets de l'invention apparaîtront plus loin dans la description.

On entendra dans tout le texte de la description par « huile présentant dans sa structure au moins un motif amide » tout composé comportant dans sa structure chimique au moins un groupement (ou fonction) amide du type : 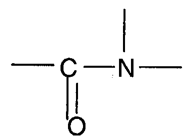 et présentant simultanément les caractéristiques suivantes :
(a) liquide à 25 °C ,
(b) insoluble ou non miscible à l'eau à 25 °C
(c) ne présente pas de propriétés émulsionnantes.

Les dérivés de la 1,7-bisphényl heptane-3,5-dione conformes à l'invention répondent à la formule (I) suivante : 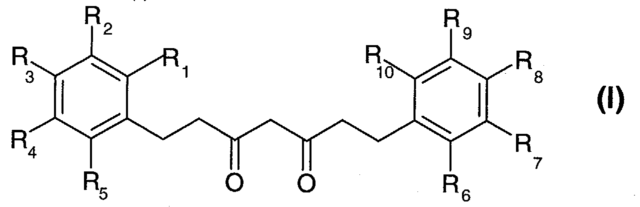 dans laquelle :
R₁ , R₂ , R₃, R₄ , R₅ , R₆, R₇, R₈, R₉, R₁₀, identiques ou différents, sont choisis parmi:
   (i) un atome d'hydrogène ;
   (ii) un radical alkyle linéaire ou ramifié en C₁-C₄ ;
   (iii) un radical OR₁₁ dans lequel R₁₁ est choisi parmi un atome d'hydrogène, un radical alkyle linéaire ou ramifié en C₁-C₄, un radical PO(OX₁)(OX₂) ou un radical SO₂(OX₃) dans lesquels X₁, X₂, et X₃, identiques ou différents, désignent un atome d'hydrogène ou un cation de métal alcalin ou NH₄⁺ ; X₁ et X₂ pouvant également désigner ensemble un cation de métal divalent,
   (iv) un radical acyle R₁₂CO dans lequel R₁₂ est choisi parmi des radicaux hydrocarbonés en C₁-C₃₀, qui sont linéaires ou ramifiés, saturés ou non saturés, hydroxylés ou non hydroxylés, carboxylés ou non carboxylés ;
   (v) un radical glycosyle ou uronyle ;
   lorsqu'un radical R₁ à R₁₀ désigne un reste OR₁₁, il peut également former avec le cycle aromatique auquel il est attaché et un radical adjacent un cycle contenant 5 ou 6 atomes ; étant entendu qu'au moins un des radicaux R₁ à R₁₀ désigne un reste OR₁₁.

Dans la formule (I) décrite ci-dessus, les radicaux alkyle peuvent désigner notamment les radicaux méthyle, éthyle, propyle, isopropyle, n-propyle, butyle, n-butyle, tert-butyle ; préférentiellement le radical alkyle désigne le radical méthyle.

Dans la formule (I) décrite ci-dessus, le radical glycosyle désigne plus particulièrement un radical glucosyle.

Dans la formule (I) décrite ci-dessus, le radical uronyle désigne plus particulièrement un radical mannuronyle ou glucuronyle.

Les composés de formule (I) sont connus en tant que tels. Les tétrahydrocurcuminoides conformes à l'invention sont obtenus par réduction de la curcumine ou synthétisés tels que décrits dans les articles « Synthesis and antibacterial activity of tetrahydrocurcuminoids ; S Venkateswarlu, M Rambabu, GV Subbaraju and S Satyanarayana ; Asian Journal of Chemistry-2000-1-141-144.» , « Synthesis of naturally occuring curcuminoids and related compounds ; U Pedersen, PB Rasmussen, SO Lawesson ; Liebigs Ann. Chem.-1985-1557-1569, les demandes de brevet JP02051595, JP02069431, JP02049747 et JP02128133, le brevet US 5,266,344 et la demande WO00/61162.

Parmi les composés de formule (I) préférentiels selon la présente invention, on peut citer :
- la 1,7-bis-(3-hydroxy-4-méthoxyphényl)heptane-3,5-dione) ou tétrahydrocurcumine (THC) de structure : 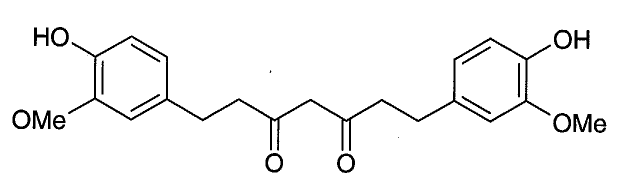
- la 1-(3-méthoxy-4-hydroxy phényl) 7-(4'-hydroxy phényl) heptane-3,5-dione ou tétrahydrodéméthoxycurcumine (THDC) de structure : 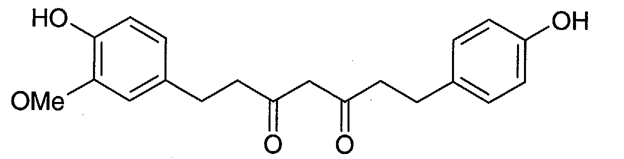
- la 1,7-bis-(4-hydroxy phényl) heptane-3,5-dione ou tétrahydrobis déméthoxycurcumine (THBDC) de structure : 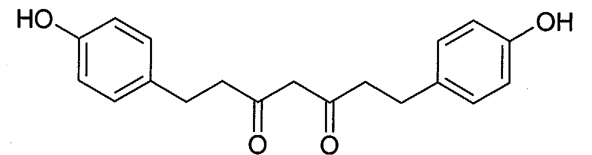
- la 1,7-bis(3,4-dihydroxyphenyl) heptane-3,5-dione de structure : 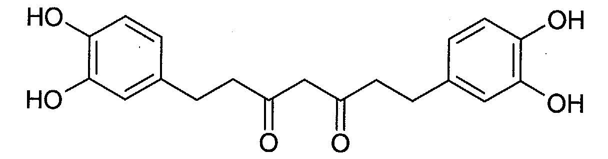
ainsi que leurs mélanges.

Une forme particulière de l'invention consiste à utiliser comme seul composé de formule (I) la 1,7-bis-(3-hydroxy-4-méthoxyphényl)heptane-3,5-dione) ou tétrahydrocurcumine (THC).

Une autre forme particulière de l'invention consiste à utiliser un mélange de composés de formule (I) constitué :
de tétrahydrocurcumine ;
de tétrahydro-déméthoxycurcumine et
de tétrahydro-bis-déméthoxycurcumine.

On utilisera encore plus particulièrement un mélange constitué :
- de 70 à 95% en poids de tétrahydrocurcumine ;
- de 4 à 25% en poids de tétrahydro-déméthoxycurcumine et
- de 0,5 à 10 % en poids de tétrahydro-bis-déméthoxycurcumine

Et encore plus particulièrement un mélange constitué :
- de 75 à 90% en poids de tétrahydrocurcumine ;
- de 8 à 20% en poids de tétrahydro-déméthoxycurcumine et
- de 1 à 5% en poids de tétrahydro-bis-déméthoxycurcumine
comme le produit décrit et synthétisé dans la demande WO00/61162 et fourni par la société SABINSA CORPORATION sous la dénomination commerciale Tetrahydrocurcuminoids CG ou Tetrahydrocurcuminoids.

De façon générale, le ou les composés de formule (I) présents dans la composition sont totalement dissous dans le support de la composition.

Les composés de formule (I) selon l'invention représentent de préférence de 0,001 à 10% en poids environ du poids total de la composition cosmétique, plus particulièrement de 0,01 à 5%, et plus préférentiellement encore de 0,1 à 5% en poids environ de ce poids.

L'huile ou les huiles présentant dans leur structure au moins un motif amide conformes à l'invention sont choisies de préférence parmi les composés de formule (II) suivante : 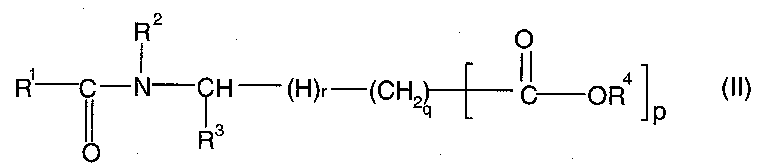 dans laquelle :
- le radical R¹ représente un radical hydrocarboné monovalent, saturé ou insaturé, aliphatique, cycloaliphatique ou cyclique, éventuellement fonctionnalisé, contenant de 1 à 30 atomes de carbone, de préférence de 1 à 22 atomes de carbone, bornes incluses ;
- les radicaux R² , R³ et R⁴ qui peuvent être identiques ou différents, représentent l'hydrogène ou des radicaux hydrocarbonés monovalents, saturés ou insaturés, aliphatiques, cycloaliphatiques ou cycliques, éventuellement fonctionnalisés, contenant de 1 à 30 atomes de carbone, de préférence de 1 à 22 atomes de carbone, bornes incluses;
- r vaut 0 ou 1 ;
- q est un entier de 0 à 2 ;
- p vaut 0 ou 1 ;
- sous réserve que :
- lorsque p = 1 alors r vaut 0 et lorsque p = 0 alors q=0 et r =1

Comme exemples de radicaux hydrocarbonés saturés aliphatiques, on peut notamment citer les radicaux alkyles, linéaires ou ramifiés, substitués ou non, en C₁-C₃₀, de préférence en C₁-C₂₂, et en particulier les radicaux méthyle, éthyle, n-propyle, isopropyle, n-butyle, isobutyle, ter.-butyle, pentyle, n-amyle, isoamyle, néopentyle, n-hexyle, n-heptyle, n-octyle, 2-éthylhexyle, tert-octyle, décyle, lauryle et octadécyle.

A titre d'exemples de radicaux hydrocarbonés saturés cycliques, on peut notamment citer les radicaux cyclopentyle et cyclohexyle, éventuellement substitués, en particulier par des radicaux alkyles.

Comme exemples de radicaux hydrocarbonés insaturés aliphatiques, on peut notamment citer les radicaux alcényles ou alcynyles, linéaires ou ramifiés, substitués ou non, en C₂-C₃₀, de préférence en C₂-C₂₂, et particulier les radicaux vinyle, allyle, oléyle et linoléyle.

Comme exemples de radicaux hydrocarbonés insaturés cycliques, on peut notamment citer les radicaux aryles tels que phényle et naphtyle, éventuellement substitués, en particulier par des alkyles, comme par exemple le radical tolyle, et à titre d'exemples de radicaux cycloaliphatiques insaturés, on peut citer plus particulièrement les radicaux benzyle et phényléthyle.

Par radicaux fonctionnalisés, on entend plus particulièrement des radicaux comportant dans leur structure chimique, tant dans la chaine principale que sur un chainon secondaire, un ou plusieurs groupements fonctionnels du type notamment esters, éthers, alcools, amines, amides et cétones, mais de préférence esters.

Les huiles amidées de formule (II) préférentielles sont choisies parmi celles où :
R¹ représente un radical alkyle en C₁-C₂₂, linéaire ou ramifiée ; un radical alcényle en C₂-C₂₂, linéaire ou ramifié ; un radical aryle ;
R² représente un atome d'hydrogène ou un groupe alkyle en C₁-C₆, linéaire ou ramifiée ;
R³ représente un atome d'hydrogène ou un groupe alkyle en C₁-C₆, linéaire ou ramifiée ;
R⁴ représente un radical alkyle en C₁-C₁₀ linéaire ou ramifié, ou un radical alcényle en C₂-C₁₀ linéaire ou ramifié ou un reste stérol.

Dans la formule (II) présentée ci-dessus, le groupement R¹(CO)- est un groupement acyle d'un acide choisi de préférence dans le groupe formé par l'acide acétique, l'acide toluique, l'acide caprique, l'acide laurique, l'acide myristique, l'acide palmitique, l'acide stéarique, l'acide béhénique, l'acide linoléique, l'acide linolénique, l'acide oléique, l'acide isostéarique, l'acide 2-éthylhexanoïque, les acides gras d'huile de coco, les acides gras d'huile de palmiste. Ces acides peuvent en outre présenter un groupe hydroxyle.

Dans la formule (II), lorsque p vaut 1, la partie -N(R²)CH(R³)(CH₂)q(CO)- de l'ester d'aminoacide est de préférence choisie parmi celles correspondant aux aminoacides suivants :
glycine, alanine, valine, leucine, isoleucine, sérine, thréonine, proline, hydroxyproline, β-alanine, N-butyl β-alanine, acide aminobutyrique, acide aminocaproïque, sarcosine, ou N-méthyl-β-alanine.

Dans la formule (II), lorsque p vaut 1, la partie des esters aminoacides correspondant au groupe OR⁴ peut être obtenue à partir des alcools choisis dans le groupe formé par le méthanol, éthanol, propanol, isopropanol, butanol, tert-butanol, isobutanol, 3-méthyl-1-butanol, 2-méthyl-1-butanol, pentanol, héxanol, cyclohéxanol, octanol, 2-éthylhéxanol, décanol, alcool laurylique, alcool myristique, alcool cétylique, alcool cétostéarylique, alcool stéarylique, alcool oléique, alcool béhénylique, alcool de jojoba, alcool 2-héxadécylique, alcool 2-octyldodécanol et alcool isostéarylique.

Les huiles présentant dans leur structure au moins une fonction amide de formule (II) conformes à l'invention sont connues en soi. Certaines sont notamment décrites avec leurs modes de préparation dans les demandes de brevets EP 1 044 676 et EP 0 928 608 de la société AJINOMOTO CO. D'autres sont connues en cosmétique comme insectifuges comme le N-acétyl N-butylaminopropionate ou le N,N-diéthyl-toluamide.

Parmi les composés de formule (II) particulièrement préférés, on peut citer :
(1) le N-acetyl N-butylaminopropionate de formule suivante : 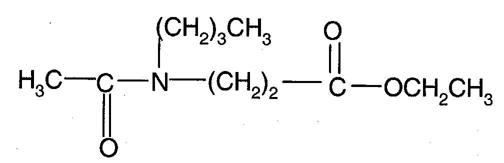 tel que le produit vendu sous la dénomination commerciale Repellent R3535 par la société MERCK ;
(2) le N-lauroylsarcosinate d'isopropyle de formule : 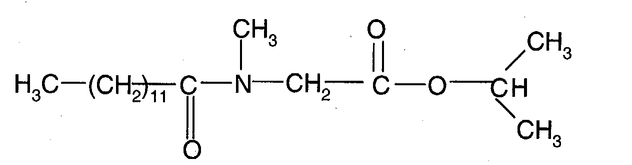 tel que le produit vendu sous le nom ELDEW SL-205 par la société Ajimoto.
(3) le N,N-diéthyl-toluamide de formule ; 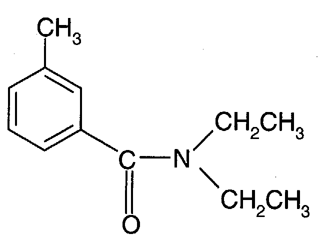 tel que le produit vendu sous le nom commercial DEET par la société Showa Denko.

L' huile ou les huiles présentant dans leur structure au moins une fonction amide telles que définies précédemment sont présentes dans les compositions selon l'invention à des concentrations allant de préférence de 0,1 à 40% en poids et plus préférentiellement de 1 à 20% en poids par rapport au poids total de la composition. L' huile ou les huiles présentant dans leur structure au moins une fonction amide seront utilisées de préférence dans une quantité suffisante permettant de solubiliser à elles seules la quantité totale de dérivé(s) de formule (I) dans la composition.

Les compositions selon l'invention sont adaptées à une application topique sur les matières kératiniques humaines en particulier la peau . Elles peuvent se présenter sous toutes les formes galéniques comportant au moins une phase grasse normalement utilisées pour ce type d'application, notamment sous forme d'une solution huileuse, d'une émulsion huile-dans-eau ou eau-dans-huile ou multiple, d'une émulsion siliconée, d'une microémulsion ou nanoémulsion, d'un gel huileux ou d'un produit anhydre liquide, pâteux ou solide.

Les compositions selon l'invention peuvent être utilisées pour prévenir ou lutter contre les effets néfastes des radiations UV et de la pollution sur les matières kératiniques humaines et plus particulièrement la peau.

Par "pollution", on entend aussi bien la pollution "extérieure", due par exemple aux particules de diesel, à l'ozone ou aux métaux lourds, que la pollution "intérieure" qui peut notamment être due aux émissions de solvants de peinture, de colles de moquette, d'isolants ou de papiers peints (tels que toluène, styrène, xylène ou benzaldéhyde), ou bien à la fumée de cigarette. Tous ces polluants sont en effet susceptibles de générer, directement ou indirectement, des radicaux libres.

De façon préférentielle, ces compositions selon l'invention contiennent en plus au moins un agent filtrant les radiations UV.

Les agents filtrant le rayonnement ultraviolet peuvent être choisis parmi les filtres UV organiques, les agents filtrant les radiations UV minéraux ou leurs mélanges.

Les filtres UV organiques conformes à l'invention peuvent être hydrosolubles, liposolubles ou insolubles dans les solvants usuels cosmétiques. Ils sont choisis notamment parmi les anthranilates ; les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469 et EP933376 ; les dérivés de la benzophénone notamment ceux décrits dans les demandes EP-A-1046391 et DE10012408 ; les dérivés de β,β'-diphénylacrylate, les dérivés de benzotriazole, les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US5,237,071, US5,166,355, GB2303549, DE19726184 et EP893119 ; les polymères filtres et silicones filtres tels que ceux décrits notamment dans la demande WO93/04665 ; les dimères dérivés d'α-alkylstyrène tels que ceux décrits dans la demande de brevet DE19855649 ; les dérivés de 4,4-diarylbutadiène tels que ceux décrits dans les demandes de brevet EP0967200 et DE19755649 et EP1133981; les hydroxybenzophénones amino substituées telles que les structures décrites dans EP1046391 et EP1133980 ; ainsi que leurs mélanges.

Comme exemples de filtres organiques, on peut citer désignés ci-dessus sous leur nom INCI :
Dérivés de l'acide para-aminobenzoique :
   PABA,
   Ethyl PABA,
   Ethyl Dihydroxypropyl PABA,
   Ethylhexyl Diméthyl PABA vendu notamment sous le nom « ESCALOL 507 » par
   ISP,
   Glyceryl PABA,
   PEG-25 PABA vendu sous le nom « UVINUL P25 » par BASF,
Dérivés salicyliques :
   Homosalate vendu sous le nom « Eusolex HMS » par Rona/EM Industries,
   Ethylhexyl Salicylate vendu sous le nom « NEO HELIOPAN OS » par Haarmann et REIMER,
   Dipropyleneglycol Salicylate vendu sous le nom « DIPSAL » par SCHER,
   TEA Salicylate, vendu sous le nom « NEO HELIOPAN TS » par Haarmann et REIMER,
Dérivés du dibenzoylméthane :
   Butyl Methoxydibenzoylmethane vendu notamment sous le nom commercial « PARSOL 1789 » par HOFFMANN LA ROCHE,
   Isopropyl Dibenzoylmethane,
Dérivés cinnamiques :
   Ethylhexyl Methoxycinnamate vendu notamment sous le nom commercial « PARSOL MCX » par HOFFMANN LA ROCHE,
   Isopropyl Methoxy cinnamate,
   Isoamyl Methoxy cinnamate vendu sous le nom commercial « NEO HELIOPAN E 1000 » par HAARMANN et REIMER,
   Cinoxate,
   DEA Methoxycinnamate,
   - Diisopropyl Methylcinnamate,
   Glyceryl Ethylhexanoate Dimethoxycinnamate
Dérivés de β,β'-diphénylacrylate :
   Octocrylene vendu notamment sous le nom commercial « UVINUL N539 » par BASF,
   Etocrylene, vendu notamment sous le nom commercial « UVINUL N35 » par BASF,
Dérivés de la benzophénone :
   Benzophenone-1 vendu sous le nom commercial « UVINUL 400 » par BASF,
   Benzophenone-2 vendu sous le nom commercial « UVINUL D50 » par BASF
   Benzophenone-3 ou Oxybenzone, vendu sous le nom commercial « UVINUL M40 » par BASF,
   Benzophenone-4 vendu sous le nom commercial « UVINUL MS40 » par BASF,
   Benzophenone-5
   Benzophenone-6 vendu sous le nom commercial « Helisorb 11 » par Norquay
   Benzophenone-8 vendu sous le nom commercial « Spectra-Sorb UV-24 » par American Cyanamid
   Benzophenone-9 vendu sous le nom commercial« UVINUL DS-49» par BASF,
   Benzophenone-12
   2-(4-diethylamino-2-hydroxybenzoyl)-benzoate de n-hexyle
Dérivés du benzylidène camphre :
   3-Benzylidene camphor fabriqué sous le nom « MEXORYL SD» par CHIMEX,
   4-Methylbenzylidene camphor vendu sous le nom « EUSOLEX 6300 » par MERCK,
   Benzylidene Camphor Sulfonic Acid fabriqué sous le nom « MEXORYL SL» par CHIMEX,
   Camphor Benzalkonium Methosulfate fabriqué sous le nom « MEXORYL SO » par CHIMEX,
   -Terephthalylidene Dicamphor Sulfonic Acid fabriqué sous le nom « MEXORYL SX » par CHIMEX,
   Polyacrylamidomethyl Benzylidene Camphor fabriqué sous le nom « MEXORYL SW » par CHIMEX,
Dérivés du phenyl benzimidazole :
   Phenylbenzimidazole Sulfonic Acid vendu notamment sous le nom commercial « EUSOLEX 232 » par MERCK,
   Disodium Phenyl Dibenzimidazole Tetra-sulfonate, vendu sous le nom commercial commercial « NEO HELIOPAN AP » par Haarmann et REIMER,
Dérivés de la triazine :
   Anisotriazine vendu sous le nom commercial «TINOSORB S » par CIBA GEIGY,
   Ethylhexyl triazone vendu notamment sous le nom commercial «UVINUL T150 » par BASF,
   Diethylhexyl Butamido Triazone vendu sous le nom commercial « UVASORB HEB » par SIGMA 3V,
Dérivés du phenyl benzotriazole :
   Drometrizole Trisiloxane vendu sous le nom « Silatrizole » par RHODIA CHIMIE ,
   Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,
Dérivés anthraniliques :
   Menthyl anthranilate vendu sous le nom commercial commercial « NEO HELIOPAN MA » par Haarmann et REIMER,
Dérivés d'imidazolines :
   Ethylhexyl Dimethoxybenzylidene Dioxoimidazoline Propionate,
Dérivés du benzalmalonate :
   Polyorganosiloxanes à fonctions benzalmalonate comme le polysilicone-15 vendu sous la dénomination commerciale « PARSOL SLX » par HOFFMANN LAROCHE et leurs mélanges.
Dérivés de 4,4-diarylbutadiène :
   -1,1-dicarboxy (2,2'-diméthyl-propyl)-4,4-diphénylbutadiène
   et leurs mélanges.

Les filtres UV organiques plus particulièrement préférés sont choisis parmi les composés suivants :
Ethylhexyl Salicylate,
Butyl Methoxydibenzoylmethane,
Ethylhexyl Methoxycinnamate,
Octocrylene,
Phenylbenzimidazole Sulfonic Acid,
Terephthalylidene Dicamphor Sulfonic Acid,.
Benzophenone-3,
Benzophenone-4,
Benzophenone-5,
2-(4-diethylamino-2-hydroxybenzoyl)-benzoate de n-hexyle
4-Methylbenzylidene camphor,
Disodium Phenyl Dibenzimidazole Tetra-sulfonate,
Anisotriazine,
Ethylhexyl triazone,
Diethylhexyl Butamido Triazone,
Methylene bis-Benzotriazolyl Tetramethylbutylphenol,
Drometrizole Trisiloxane,
Polysilicone-15
1,1-dicarboxy (2,2'-diméthyl-propyl)-4,4-diphénylbutadiène
et leurs mélanges.

Les agents filtrant minéraux sont généralement des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A-0518772 et EP-A-0518773.

Les agents filtrants les radiations conformes à l'invention sont généralement présents dans les compositions selon l'invention dans des proportions allant de 0,1 à 20% en poids par rapport au poids total de la composition, et de préférence allant de 0,2 à 15% en poids par rapport au poids total de la composition.

Les compositions selon l'invention peuvent également contenir des agents de bronzage et/ou de brunissage artificiels de la peau (agents autobronzants), et plus particulièrement la dihydroxyacétone (DHA).

Un autre objet de l'invention consiste à l'utilisation d'une composition selon l'invention comme produit autobronzant et/ou pour le brunissage artificiel de la peau.

Les compositions selon l'invention peuvent notamment être destinées à prévenir la formation des radicaux libres au contact de la peau et/ou à neutraliser les radicaux libres déjà formés.

Les compositions selon l'invention peuvent contenir en plus un ou plusieurs agents anti-pollution et/ou agent anti-radicalaire.

Par l'expression "agent anti-pollution", on entend tout composé capable de piéger l'ozone, les composés aromatiques mono- ou polycycliques tels que le benzopyrène et/ou les métaux lourds tels que le cobalt, le mercure, le cadmium et/ou le nickel. Par "agent anti-radicalaire", on entend tout composé capable de piéger les radicaux libres.

Comme agents piégeurs d'ozone utilisables dans la composition selon l'invention, on peut citer en particulier les phénols et polyphénols, en particulier les tannins, l'acide ellagique et l'acide tannique ; l'épigallocatéchine et les extraits naturels en contenant ; les extraits de feuilles d'olivier ; les extraits de thé, en particulier de thé vert ; les anthocyanes ; les flavonoïdes, en particulier la rutine et l'alpha glycosyl rutine; les extraits de romarin ; les acides phénols, en particulier l'acide chorogénique ; les stilbènes, en particulier le resvératrol ; les dérivés d'acides aminés soufrés, en particulier la S-carboxyméthylcystéine ; l'ergothionéine ; la N-acétylcystéine ; des chélatants comme la N,N'-bis-(3,4,5-triméthoxybenzyl)éthylènediamine ou l'un de ses sels, complexes métalliques ou esters ; des caroténoïdes tels que la crocétine ; et des matières premières diverses comme le mélange d'arginine, ribonucléate d'histidine, mannitol, adénosinetriphosphate, pyridoxine, phénylalanine, tyrosine et ARN hydrolysé commercialisé par les Laboratoires Sérobiologiques sous la dénomination commerciale CPP LS 2633-12F®, la fraction hydrosoluble de maïs commercialisée par la société SOLABIA sous la dénomination commerciale Phytovityl®, le mélange d'extrait de fumeterre et d'extrait de citron commercialisé sous la dénomination Unicotrozon C-49® par la société Induchem, et le mélange d'extraits de ginseng, de pomme, de pêche, de blé et d'orge vendu par la société PROVITAL sous la dénomination commerciale Pronalen Bioprotect®.

Comme agents piégeurs de composés aromatiques mono- ou polycycliques utilisables dans la composition selon l'invention, on peut citer en particulier les tannins tels que l'acide ellagique ; les dérivés indoles, en particulier l'indol-3-carbinol ; les extraits de thé en particulier de thé vert, les extraits de Jacinthe d'eau ou eichornia crassipes ; et la fraction hydrosoluble de maïs commercialisée par la société SOLABIA sous la dénomination commerciale Phytovityl®.

Enfin, comme agents piégeurs de métaux lourds utilisables dans la composition selon l'invention, on peut citer en particulier les agents chélatants tels que l'EDTA, le sel pentasodique d'éthylènediamine tétraméthylène phosphonique, et la N,N'-bis-(3,4,5-triméthoxybenzyl)éthylènediamine ou l'un de ses sels, complexes métalliques ou esters ; l'acide phytique ; les dérivés de chitosan ; les extraits de thé, en particulier de thé vert ; les tannins tels que l'acide ellagique ; les acides aminés soufrés tels que la cystéine ; les extraits de Jacinthe d'eau (Eichornia crassipes) ; et la fraction hydrosoluble de maïs commercialisée par la société SOLABIA sous la dénomination commerciale Phytovityl®.

Les agents anti-radicalaires utilisables dans la composition selon l'invention comprennent, outre certains agents anti-pollution mentionnés précédemment, la vitamine E et ses dérivés tels que l'acétate de tocophéryle ; les bioflavonoïdes ; le co-enzyme Q10 ou ubiquinone ; certaines enzymes comme la catalase, le superoxyde dismutase, la lactoperoxydase, le glutathion peroxydase et les quinones réductases ; le glutathion ; le benzylidène camphre ; les benzylcyclanones ; les naphtalénones substituées ; les pidolates ; le phytantriol ; le gamma-oryzanol ; les lignanes tels que l'acide nordihydroguaiaretique (NDGA) ; et la mélatonine.

Les compositions selon l'invention peuvent également être utilisées pour traiter les cheveux endommagés par les effets néfastes des radiations UV et de la pollution. Elles peuvent contenir en plus un ou plusieurs inhibiteurs de lysyl et/ou prolyl hydroxylase

Des exemples préférés d'inhibiteurs de lysyl et/ou propyl hydroxylase utilisables dans la composition selon la présente invention sont le 2,4-diamino-pyrimidine 3-oxyde ou 2,4-DPO décrit dans la demande de brevet WO 96/09048 et le 2,4-diamino-6-pipéridino pyrimidine 3-oxyde ou "Minoxidil" décrit dans les brevets US-4,139,619 et US-4,596,812.

Les compositions selon l'invention peuvent être utilisées pour traiter les peaux endommagées par les effets néfastes des radiations UV et de la pollution et notamment traiter les peaux desséchées, craquelées, irritées ou brûlées par le soleil ou la pollution. Ces compositions peuvent contenir en plus un ou plusieurs plusieurs actifs choisis parmi les agents desquamants ; les agents hydratants ; les agents anti-inflammatoires ; les agents agissant sur le métabolisme énergétique des cellules ou leurs mélanges.

Par "agent desquamant", on entend tout composé capable d'agir :
- soit directement sur la desquamation en favorisant l'exfoliation, tel que les β-hydroxyacides, en particulier l'acide salicylique et ses dérivés (dont l'acide n-octanoyl 5-salicylique) ; les α-hydroxyacides, tels que les acides glycolique, citrique, lactique, tartrique, malique ou mandélique ; l'urée ; l'acide gentisique ; les oligofucoses ; l'acide cinnamique ; l'extrait de Sophora japonica ; le resvératrol ;
- soit sur les enzymes impliquées dans la desquamation ou la dégradation des cornéodesmosomes, les glycosidases, la stratum corneum chymotryptic enzym (SCCE) voire d'autres protéases (trypsine, chymotrypsine-like). On peut citer les agents chélatant des sels minéraux: l'EDTA ; l'acide N-acyl-N,N',N' éthylène diaminetriacétique ; les composés aminosulfoniques et en particulier l'acide (N-2 hydroxyéthylpiperazine-N-2-éthane) sulfonique (HEPES) ; les dérivés de l'acide 2-oxothiazolidine-4-carboxylique (procystéine) ; l'acide méthylglycine diacétique et ses sels (TRILON M de BASF) ; les dérivés d'acides alpha aminés de type glycine (tels que décrits dans EP-0 852 949) ; le miel ; les dérivés de sucre tels que l'O-octanoyl-6-D-maltose et la N-acétyl glucosamine.

Par "agent hydratant", on entend :
- soit un composé agissant sur la fonction barrière, en vue de maintenir l'hydratation du stratum corneum, ou un composé occlusif. On peut citer les céramides, la vaseline et la lanoline, les composés à base de sphingoïde, les lécithines, les glycosphingolipides, les phospholipides, le cholestérol et ses dérivés, les phytostérols (stigmastérol, β-sitostérol, campestérol), les acides gras essentiels, le 1-2 diacylglycérol, la 4-chromanone, les triterpènes pentacycliques tel que l'acide ursolique
- soit un composé augmentant directement la teneur en eau du stratum corneum, tel que le tréhalose et ses dérivés, l'acide hyaluronique et ses dérivés, le glycérol, le pentanediol, le pidolate de sodium, la sérine, le xylitol, le lactate de sodium, le polyacrylate de glycérol, l'ectoïne et ses dérivés, le chitosane, les oligo- et polysaccharides, les carbonates cycliques, l'acide N-lauroyl pyrrolidone carboxylique, et la N-α-benzoyl-L-arginine ;
- soit un composé activant les glandes sébacées tel que les dérivés stéroïdiens (dont la DHEA) et la vitamine D et ses dérivés.

Par "agent anti-inflammatoire", on entend tout composé susceptible d'inhiber les principales enzymes impliquées dans le processus inflammatoire (cascade de l'acide arachidonique), à savoir : les phospholipases A2 (PLA2) ; les lipoxygénases (Lox) ; et les prostaglandines humaines synthétases (PGHS).

Parmi les matières premières efficaces pour inhiber au moins une de ces enzymes, on peut citer de façon non limitative les actifs suivants :
- Les triterpènes pentacycliques et les extraits de plante (ex : Glycyrrhiza glabra) en contenant comme l'acide β-glycyrrhétinique et ses sels et/ou ses dérivés (l'acide glycyrrhetique monoglucuronide, le stearyl glycyrrhetinate, l'acide 3- stéaroyloxy glycyrrhetique), l'acide ursolique et ses sels, l'acide oléanolique et ses sels, l'acide bétulinique et ses sels, l'extrait de Paeonia suffruticosa et / ou lactiflora, l'huile de calophilum, les sels de l'acide salicylique et en particulier le salicylate de zinc, les phycosaccharides anti-inflammatoires (hydrolysed algin ou hydrolysed algin and zinc sulfate) de la société Codif, la phlorogine (laminaria saccharina) de Secma, l'huile de Canola, de Tamanu, de calophyllum, l'α-bisabolol et les extraits de camomille, l'allantoine, les huiles insaturées en oméga 3 telles que les huiles de rosier musca, de cassis, d'ecchium, de poisson, l'omega plancton (plancton extract) de Secma, le lipacide C8G ( capriloyl glycine) de Seppic, le Seppicalm VG (sodium palmitoylproline and nymphea alba) de Seppic, l'extrait d'epilobe, l'extrait du pygeum, le Soothex ( extrait de boswellia serrata) de Quest, le phytoplenolin (extrait de centipeda cunnighami) de Bio-Botanica, l' hélioxine (extrait d'hélianthus annuus) de Silab, la Sensiline (linum usitatissimum) de Silab, les tocotrienols, les extraits de Cola nitida, le piperonal, l'extrait de clou de girofle, l'extrait d'épilobe (Epilobium Angustifolium) l'aloe vera, la bacocalmine (extrait de bacopa moniera) de Séderma, les phytostérols, la cortisone, l'hydrocortisone, l'indometacine et la béta methasone.

Les actifs agissant sur le métabolisme énergétique des cellules sont ceux qui agissent sur le métabolisme énergétique cutané tel que, par exemple, et de façon non limitative, la synthèse d'ATP, ceux qui interviennent sur la chaîne respiratoire de la cellule ou sur les réserves énergétiques. On peut citer le Coenzyme Q10 (ubiquinone), le cytochrome C, la créatine ou encore la phosphocréatine.

Les compositions selon l'invention peuvent être utilisées pour prévenir et/ou traiter les signes du vieillissement cutané, en particulier du photo-vieillissement de la peau et notamment la perte de fermeté et/ou d'élasticité de la peau.

Elles peuvent contenir en plus un ou plusieurs actifs choisis dans le groupe constitué par les agents anti-glycation ; les inhibiteurs de NO-synthase ; les agents stimulant la synthèse de macromolécules dermiques ou épidermiques et/ou empêchant leur dégradation ; les agents stimulant la prolifération des fibroblastes ; les agent myorelaxants ; les agents tenseurs.

Par "agent anti-glycation", on entend un composé prévenant et/ou diminuant la glycation des protéines de la peau, en particulier des protéines du derme telles que le collagène.

Des exemples d'agents anti-glycation sont les extraits végétaux de la famille des Ericaceae, tels qu'un extrait de myrtille (Vaccinium angusfifollium) ; l'ergothionéine et ses dérivés ; et les hydroxystilbènes et leurs dérivés, tels que le resvératrol et le 3,3', 5,5'-tétrahydroxystilbène. Ces agents anti-glycation sont décrits dans les demandes FR 99/16166, FR 00/08158, FR 99/09267 et FR 99/16168, respectivement. Le resvératrol est particulièrement préféré pour une utilisation dans cette invention.

Des exemples d'inhibiteurs de NO-synthase convenant à une utilisation dans la présente invention comprennent notamment un extrait de végétal de l'espèce Vitis vinifera qui est notamment commercialisé par la société Euromed sous la dénomination Leucocyanidines de raisins extra, ou encore par la société Indena sous la dénomination Leucoselect®, ou enfin par la société Hansen sous la dénomination Extrait de marc de raisin ; un extrait de végétal de l'espèce Olea europaea qui est de préférence obtenu à partir de feuilles d'olivier et est notamment commercialisé par la société VINYALS sous forme d'extrait sec, ou par la société Biologia & Technologia sous la dénomination commerciale Eurol BT ; et un extrait d'un végétal de l'espèce Gingko biloba qui est de préférence un extrait aqueux sec de ce végétal vendu par la société Beaufour sous le nom commercial Ginkgo biloba extrait standard.

Parmi les actifs stimulant les macromolécules du derme, on peut citer ceux qui agissent :
- soit sur la synthèse du collagène, tels que les extraits de Centella asiatica ; les asiaticosides et dérivés ; les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine® ; et les hormones végétales telles que les auxines.
- soit sur la synthèse d'élastine, tels que l'extrait de Saccharomyces Cerivisiae commercialisé par la société LSN sous la dénomination commerciale Cytovitin® ; et l'extrait d'algue Macrocystis pyrifera commercialisé par la société SECMA sous la dénomination commerciale Kelpadelie® ;
- soit sur la synthèse des glycosaminoglycanes, tels que le produit de fermentation du lait par lactobacillus vulgaris, commercialisé par la société BROOKS sous la dénomination commerciale Biomin yogourth® ; l'extrait d'algue brune Padina pavonica commercialisé par la société ALBAN MÜLLER sous la dénomination commerciale HSP3® ; et l'extrait de Saccharomyces cerevisiae disponible notamment auprès de la société SILAB sous la dénomination commerciale Firmalift® ou auprès de la société LSN sous la dénomination commerciale Cytovitin® ;
- soit sur la synthèse de la fibronectine, tels que l'extrait de zooplancton Salina commercialisé par la société SEPORGA sous la dénomination commerciale GP4G® ;
   l'extrait de levure disponible notamment auprès de la société ALBAN MÜLLER sous la dénomination commerciale Drieline® ; et le palmitoyl pentapeptide commercialisé par la société SEDERMA sous la dénomination commerciale Matrixil® ;
- soit sur l'inhibition métalloprotéinases (MMP) telles que plus particulièrement les MMP 1, 2, 3, 9 . On peut citer : les rétinoïdes et dérivés, les isoflavonoïdes, les oligopeptides et les lipopeptides, les lipoaminoacides, l'extrait de malt commercialisé par la société COLETICA sous la dénomination commerciale Collalift® ; les extraits de myrtille ou de romarin ; le lycopène ; les isoflavones, leurs dérivés ou les extraits végétaux en contenant, en particulier les extraits de soja (commercialisé par exemple par la société ICHIMARU PHARCOS sous la dénomination commerciale Flavostérone SB®), de trèfle rouge, de lin, de kakkon ou de sauge ;
- soit sur l'inhibition des sérine protéases telles que l'élastase leucocytaire ou la cathepsine G. On peut citer: l'extrait peptidique de graines de légumineuse (Pisum sativum) commercialisé par la société LSN sous la dénomination commerciale Parelastyl® ; les héparinoïdes ; et les pseudodipeptides.

Parmi les actifs stimulant les macromolécules épidermiques, telles que la fillagrine et les kératines, on peut citer notamment l'extrait de lupin commercialisé par la société SILAB sous la dénomination commerciale Structurine® ; l'extrait de bourgeons de hêtre Fagus sylvatica commercialisé par la société GATTEFOSSE sous la dénomination commerciale Gatuline® ; et l'extrait de zooplancton Salina commercialisé par la société SEPORGA sous la dénomination commerciale GP4G®.

Les agents stimulant la prolifération des fibroblastes utilisables dans la composition selon l'invention peuvent par exemple être choisis parmi les protéines ou polypeptides végétaux, extraits notamment du soja (par exemple un extrait de soja commercialisé par la société LSN sous la dénomination Eleseryl SH-VEG 8® ou commercialisé par la société SILAB sous la dénomination commerciale Raffermine®) ; et les hormones végétales telles que les giberellines et les cytokinines.

Les agents stimulant la prolifération des kératinocytes, utilisables dans la composition selon l'invention, comprennent notamment les rétinoïdes tels que le rétinol et ses esters, dont le palmitate de rétinyle ; les extraits de tourteaux de noix commercialisés par la société GATTEFOSSE ; et les extraits de Solanum tuberosum commercialisés par la société SEDERMA.

Les agents stimulant la différenciation des kératinocytes comprennent par exemple les minéraux tels que le calcium ; l'extrait de lupin commercialisé par la société SILAB sous la dénomination commerciale Photopréventine®; le betasitosteryl sulfate de sodium commercialisé par la société SEPORGA sous la dénomination commerciale Phytocohésine® ; et l'extrait de maïs commercialisé par la société SOLABIA sous la dénomination commerciale Phytovityl®.

Les agents myorelaxants utilisables dans la composition selon l'invention comprennent les inhibiteurs calciques tels que l'alvérine et ses sels, les ouvreurs de canaux chlore tel que le Diazepam, et les inhibiteurs de catécholamines et d'acétylcholine tels que l'hexapeptide argireline R commercialisé par la société LIPOTEC.

Parmi les agents tenseurs utilisables dans la composition selon la présente invention, on peut citer notamment :
(1) les latex de polyuréthanne ou les latex acrylique-silicone, en particulier ceux décrits dans la demande de brevet EP-1038519, tels qu'un polydiméthyl siloxane greffé propylthio(polyacrylate de méthyle), propylthio(polyméthacrylate de méthyle) et propylthio(polyacide méthacrylique), ou encore un polydiméthyl siloxane greffé propylthio(polyméthacrylate d'isobutyle) et propylthio(polyacide méthacrylique). De tels polymères siliconés greffés sont notamment vendus par la Société 3M sous les dénominations commerciales VS 80, VS 70 ou LO21,
(2) les protéines végétales de soja ou de blé, et/ou
(3) les silicates de sodium et magnésium (Laponites) et/ou
(4) les particules colloïdales de charges inorganiques, en particulier de silice.

Les compositions selon l'invention peuvent également être utilisées pour prévenir ou réduire la formation de taches pigmentaires et/ou pour blanchir ou dépigmenter la peau. Elles peuvent contenir en plus un ou plusieurs agents dépigmentants et/ou des agents pro-pigmentants.

Les agents dépigmentants susceptibles d'être incorporés dans la composition selon la présente invention comprennent par exemple les composés suivants : l'acide kojique ; l'acide ellagique ; l'arbutine et ses dérivés tels que ceux décrits dans les demandes EP-895 779 et EP-524 109 ; l'hydroquinone ; les dérivés d'aminophénol tels que ceux décrits dans les demandes WO 99/10318 et WO 99/32077, et en particulier le N-cholestéryloxycarbonyl-para-aminophénol et le N-éthyloxycarbonyl-para-aminophénol ; les dérivés d'iminophénol, en particulier ceux décrits dans la demande WO 99/22707 ; l'acide L-2-oxothiazolidine-4-carboxylique ou procystéine, ainsi que ses sels et esters ; et les extraits de plantes, en particulier de réglisse, de saxifraga, de goyave, de poire, de raisin, de mûrier et de scutellaire, sans que cette liste soit limitative.

Comme agent pro-pigmentant, on peut citer l'extrait de pimprenelle (Sanguisorba officinalis) commercialisé par la société MARUZEN et les extraits de chrysanthème (Chrysanthemum morifolium).

Les compositions conformes à la présente invention peuvent comprendre en outre des adjuvants cosmétiques classiques notamment choisis parmi les corps gras, les solvants organiques, les épaississants ioniques ou non ioniques, les adoucissants, les humectants, les opacifiants, les stabilisants, les émollients, les silicones, les agents anti-mousse, les agents répulsifs contre les insectes, les parfums, les conservateurs, les tensioactifs anioniques, cationiques, non-ioniques, zwitterioniques ou amphotères, les antagonistes de substance P, les antagonistes de substance CGRP, les charges, les polymères, les propulseurs, les agents alcalinisants ou acidifiants ou tout autre ingrédient habituellement utilisé dans le domaine cosmétique et/ou dermatologique.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (paraffine); végétales (huile d'amande douce, de macadamia, de pépin de cassis, de jojoba) ; synthétiques comme le perhydrosqualène, les alcools, les acides ou les esters gras (comme le benzoate d'alcools en C₁₂-C₁₅ vendu sous la dénomination commerciale « Finsolv TN » par la société WITCO, le palmitate d'octyle, le lanolate d'isopropyle, les triglycérides dont ceux des acides caprique/caprylique), les esters et éthers gras oxyéthylénés ou oxypropylénés; les huiles siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Parmi les solvants organiques, on peut citer les alcools et polyols inférieurs. Ces derniers peuvent être choisis parmi les glycols et les éthers de glycol comme l'éthylène glycol, le propylène glycol, le butylène glycol, le dipropylène glycol ou le diéthylène glycol.

Les épaississants peuvent être choisis notamment parmi les acides polyacryliques réticulés, les polymères d'acide acrylamidométhylpropane sufonique (AMPS) comme le polyacrylamide/isoparaffine/laureth-7 (Sepigel 305), l'acide stéarique, les alcools gras, les gommes de xanthane, les gommes de guar et celluloses modifiées ou non telles que la gomme de guar hydroxypropylée, la méthylhydroxyéthylcellulose et l'hydroxypropylméthyl cellulose.

Bien entendu, l'homme de l'art veillera à choisir le ou les éventuels composés complémentaires cités ci-dessus et/ou leurs quantités de manière telle que les propriétés avantageuses attachées intrinsèquement à l'association conforme à l'invention ne soient pas, ou substantiellement pas, altérées par la ou les adjonctions envisagées.

Les compositions selon l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, ou sous la forme d'un gel ou d'un gel crème, sous la forme d'une lotion, de poudre, de bâtonnet solide et éventuellement être conditionnée en aérosol et se présenter sous forme de mousse ou de spray.

De préférence, les compositions selon l'invention se présentent sous la forme d'une émulsion huile-dans-eau ou eau-dans huile.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

Lorsque la composition cosmétique selon l'invention est utilisée pour le soin de l'épiderme humain, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de poudre, de mousse aérosol ou de spray.

Lorsque la composition cosmétique selon l'invention est utilisée pour le soin des cheveux, elle peut se présenter sous forme de shampooing, de lotion, de gel, d'émulsion, de dispersion vésiculaire non ionique et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de soin des ongles, des lèvres, des cils, des sourcils, elle peut se présenter sous forme solide ou pâteuse, anhydre ou aqueuse, comme des émulsions huile dans eau ou eau dans huile, des dispersions vésiculaires non ioniques ou encore des suspensions, sous forme de bâtonnet solide.

Des exemples concrets, mais nullement limitatifs, illustrant l'invention vont maintenant être donnés.

### Test de solubilité

On compare 2 huiles amidées conformes à l'invention à 13 huiles classiques utilisées en cosmétique. On étudie leur pouvoir solubilisant sur un mélange de tétrahydrocurcumunoides de formule (I) : ie le produit commercial Tetrahydrocurcuminoids CG de Sabinsa tel que défini précédemment.

### Mode opératoire :

On pèse 1g de mélange de tétrahydrocurcuminoides et on complète à 10g avec l'huile étudiée. On chauffe pendant 15 minutes dans un bain-marie à 80°C. On laisse refroidir 24 heures à température ambiante et on réalise une observation macroscopique.

| **Composition huileuse** | **Huile testée** | **Solubilité après 24 heures à température ambiante** |
|---|---|---|
| 1 (invention) | N-éthyl N-butylacétylaminopropionate | **Pas de cristaux** |
| 2 (invention) | N-lauroylsarcosinate d'isopropyle | **Pas de cristaux** |
| 3 comparatif | Huile de vaseline | Cristaux + dépôt |
| 4 comparatif | Dicaprylyl maleate | Cristaux + dépôt |
| 5 comparatif | C₁₂-₁₅Alkyl benzoate | Cristaux + dépôt |
| 6 comparatif | Cire liquide de Jojoba | Cristaux + dépôt |
| 7 comparatif | Huile d'amande d'abricot raffinée | Cristaux + dépôt |
| 8 comparatif | Isohexadécane | Cristaux + dépôt |
| 9 comparatif | Carbonate de dicaprylyle | Cristaux + dépôt |
| 10 Comparatif | Triglycerides d'acides caprylique/caprique | Cristaux + dépôt |
| 11 comparatif | Triméllitate de tridécyle | Cristaux + dépôt |
| 12 comparatif | Cyclopenta diméthyl siloxane | Cristaux + dépôt |
| 13 comparatif | Malate de dioctyle | Cristaux + dépôt |
| 14 comparatif | Palmitate d'isopropyle | Cristaux + dépôt |
| 15 comparatif | Isononanoate d'isononyle | Cristaux + dépôt |

Seules les huiles comportant dans leur structure un motif amide conforme à l'invention : N-lauroyl sarcosinate d'isopropyle et le N-éthyl N-butylaminopropionate permettent de dissoudre totalement le mélange de tetrahydrocurcuminoïdes.

### Stabilité en formulation

Le mélange de tétrahydrocurcuminoides de formule (I) (Tetrahydrocurcuminoids CG de Sabinsa) a été mis en formulation à 0.5% dans trois émulsions H/E en faisant varier la nature du solvant utilisé pour solubiliser ledit mélange. Les compositions des formules testées sont décrites ci dessous et les quantités sont exprimées en grammes.

| **COMPOSITIONS TESTEES** | **A (hors Invention)** | **B (invention)** | **C (invention)** |
|---|---|---|---|
| Mélange d'alcool cétylstéarylique et d'alcool cétylstéarylique oxyéthyléné (33 OE) 80/20 | 7 | 7 | 7 |
| Mélange de mono et distéarate de glycérol | 2 | 2 | 2 |
| Alcool cétylique | 1.5 | 1.5 | 1.5 |
| Polydiméthyl siloxane | 1 | 1 | 1 |
| Huile de vaseline | 15 | 15 | 15 |
| Butyl Methoxydibenzoylmethane | 3 | 3 | 3 |
| Glycérine | 20 | 20 | 20 |
| Conservateurs | 1.2 | 1.2 | 1.2 |
| Mélange de tétrahydrocurcuminoïdes de formule (I) (Tetrahydrocurcuminoids CG de Sabinsa) | 0.5 | 0.5 | 0.5 |
| Malate de dioctyle | 7 | | |
| N-lauroylsarcosinate d'isopropyle | | 7 | |
| N-éthyl N-butylacétylaminopropionate | | | 7 |
| Eau déminéralisée qsp | 100 g | 100 g | 100 g |

### Méthode d'évaluation de la stabilité des formules

Au bout de 1 mois de conservation à température ambiante, la stabilité de ces formules est évaluée au niveau microscopique, pour détecter la présence de cristaux de tétrahydrocurcuminoïdes.

| **COMPOSITIONS TESTEES** | **A (hors invention)** | **B (invention)** | **C (invention)** |
|---|---|---|---|
| Aspect microscopique | Présence de nombreux cristaux | Pas de cristaux | Pas de cristaux |

Au sein d'une émulsion, le fait de solubiliser le mélange de tétrahydrocurcuminoïdes dans une huile présentant dans sa structure au moins un motif amide conforme à l'invention permet d'obtenir une bonne stabilité au cours du temps.

## Revendications

1. Composition cosmétique ou dermatologique contenant un support comprenant au moins une phase grasse **caractérisée par le fait qu'**elle contient
- au moins un dérivé ou un mélange de dérivés de la 1,7-bisphényl heptane-3,5-dione de formule (I) suivante : 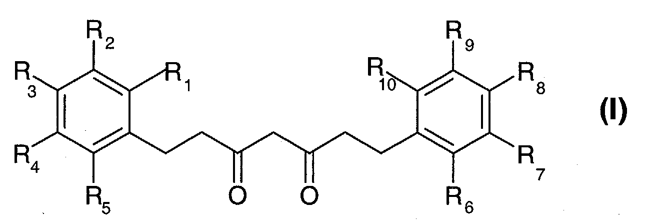 dans laquelle :
R₁ , R₂ , R₃, R₄ , R₅ , R₆, R₇, R₈, R₉, R₁₀, identiques ou différents, sont choisis parmi :
(i) un atome d'hydrogène ;
(ii) un radical alkyle linéaire ou ramifié en C₁-C₄ ;
(iii) un radical OR₁₁ dans lequel R₁₁ est choisi parmi un atome d'hydrogène, un radical alkyle linéaire ou ramifié en C₁-C₄, un radical PO (OX₁)(OX₂) ou un radical SO₂(OX₃) dans lesquels, X₁, X₂, et X₃, identiques ou différents, désignent un atome d'hydrogène ou un cation de métal alcalin ou NH₄⁺ , X₁ et X₂ pouvant également désigner ensemble un cation de métal divalent,
(iv) un radical acyle R₁₂CO dans lequel R₁₂ est choisi parmi des radicaux hydrocarbonés en C₁-C₃₀, qui sont linéaires ou ramifiés, saturés ou non saturés, hydroxylés ou non hydroxylés, carboxylés ou non carboxylés ;
(v) un radical glycosyle ou uronyle ;
lorsqu'un radical R₁ à R₁₀ désigne un reste OR₁₁, il peut également former avec le cycle aromatique auquel il est attaché et un radical adjacent un cycle contenant 5 ou 6 atomes ; étant entendu qu'au moins un des radicaux R₁ à R₁₀ désigne un reste OR₁₁ ;
- au moins une huile présentant dans sa structure au moins un motif amide.

2. Composition selon la revendication 1, où le ou les composés de formule (I) sont choisis dans le groupe constitué par :
- la 1,7-bis-(3-méthoxy-4-hydroxy phényl)heptane-3,5-dione) ou tétrahydrocurcumine (THC) de structure : 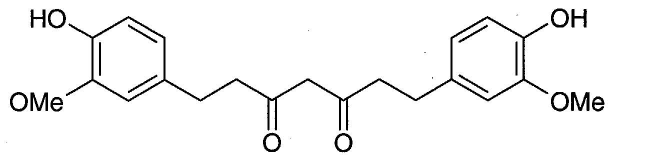
- la 1-(3-méthoxy-4-hydroxy phényl) 7-(4'-hydroxy phényl) heptane-3,5-dione ou tétrahydrodéméthoxycurcumine (THDC) de structure : 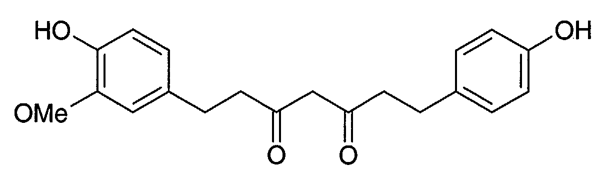
- la 1,7-bis-(4-hydroxy phényl) heptane-3,5-dione ou tétrahydrobis déméthoxycurcumine (THBDC) de structure : 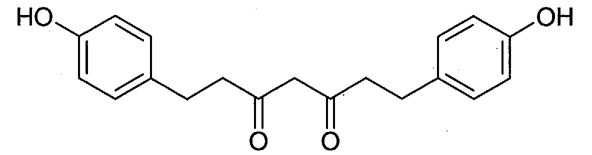
- la 1,7-bis(3,4-dihydroxyphenyl) heptane-3,5-dione de structure : 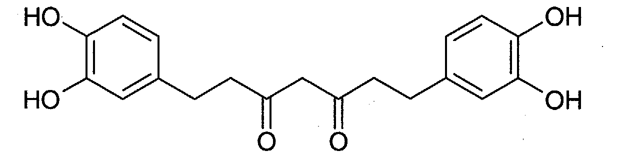
ainsi que leurs mélanges.

3. Composition selon la revendication 2, **caractérisée par le fait qu'**elle contient, comme seul composé de formule (I), la 1,7-bis-(3-méthoxy-4-hydroxyphényl)heptane-3,5-dione) ou tétrahydrocurcumine (THC).

4. Composition selon la revendication 1 ou 2, **caractérisée par le fait qu'**elle contient un mélange de composés de formule (I) constitué :
(a) de tétrahydrocurcumine ;
(b) de tétrahydro-déméthoxycurcumine et
(c) de tétrahydro-bis-déméthoxycurcumine.

5. Composition selon la revendication 4, **caractérisée par le fait qu'**elle contient un mélange de composés de formule (I) constitué :
- de 70 à 95% en poids de tétrahydrocurcumine ;
- de 4 à 25% en poids de tétrahydro-déméthoxycurcumine et
- de 0,5 à 10 % en poids de tétrahydro-bis-déméthoxycurcumine.

6. Composition selon la revendication 5, **caractérisée qu'**elle contient un mélange de composés de formule (I) constitué :
- de 75 à 90% en poids de tétrahydrocurcumine ;
- de 8 à 20% en poids de tétrahydro-déméthoxycurcumine et
- de 1 à 5 % en poids de tétrahydro-bis-déméthoxycurcumine.

7. Composition selon l'une quelconque des revendications 1 à 6, **caractérisée par le fait que** le ou les composés de formule (I) sont totalement dissous dans le support de la composition.

8. Composition selon l'une quelconque des revendications 1 à 7, **caractérisée par le fait que** le ou les composés de formule (I) représentent de 0,001 à 10% en poids du poids total de la composition cosmétique, plus particulièrement de 0,01 à 5%, et encore plus préférentiellement de 0,1 à 5% en poids environ de ce poids.

9. Composition selon l'une quelconque des revendications 1 à 8, **caractérisée par le fait que** les huiles présentant dans sa structure au moins un motif amide sont choisies parmi les composés de formule (II) suivante : 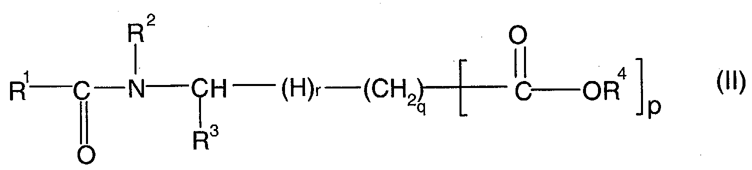 dans laquelle :
- le radical R¹ représente un radical hydrocarboné monovalent, saturé ou insaturé, aliphatique, cycloaliphatique ou cyclique, éventuellement fonctionnalisé, contenant de 1 à 30 atomes de carbone, de préférence de 1 à 22 atomes de carbone, bornes incluses ;
- les radicaux R² , R³ et R⁴ qui peuvent être identiques ou différents, représentent l'hydrogène ou des radicaux hydrocarbonés monovalents, saturés ou insaturés, aliphatiques, cycloaliphatiques ou cycliques, éventuellement fonctionnalisés, contenant de 1 à 30 atomes de carbone, de préférence de 1 à 22 atomes de carbone, bornes incluses;
- r vaut 0 ou 1 ;
- q est un entier de 0 à 2 ;
- p vaut 0 ou 1 ;
- sous réserve que :
- lorsque p = 1 alors r vaut 0 et lorsque p = 0 alors q=0 et r =1

10. Composition selon la revendication 9 où les huiles de formule (II) sont choisies parmi celles où :
R¹ représente un radical alkyle en C₁-C₂₂, linéaire ou ramifiée ; un radical alcényle en C₂-C₂₂, linéaire ou ramifié ; un radical aryle ;
R² représente un atome d'hydrogène ou un groupe alkyle en C₁-C₆, linéaire ou ramifiée ;
R³ représente un atome d'hydrogène ou un groupe alkyle en C₁-C₆, linéaire ou ramifiée ;
R⁴ représente un radical alkyle en C₁-C₁₀ linéaire ou ramifié, ou un radical alcényle en C₂-C₁₀ linéaire ou ramifié ou un reste stérol.

11. Composition selon la revendication 10 où les huiles de formule (II) sont choisies parmi celles où le groupement R¹(CO)- est un groupement acyle d'un acide choisi dans le groupe formé par l'acide acétique, l'acide toluique, l'acide caprique, l'acide laurique, l'acide myristique, l'acide palmitique, l'acide stéarique, l'acide béhénique, l'acide linoléique, l'acide linolénique, l'acide oléique, l'acide isostéarique, l'acide 2-éthylhexanoïque, les acides gras d'huile de coco, les acides gras d'huile de palmiste ; ces acides pouvant en outre présenter un groupe hydroxyle.

12. Composition selon la revendication 10 ou 11, où les huiles de formule (II) sont choisies parmi celles où p vaut 1 et la partie -N(R²)CH(R³)(CH₂)q(CO)- de l'ester d'aminoacide est choisie parmi celles correspondant aux aminoacides suivants :
glycine, alanine, valine, leucine, isoleucine, sérine, thréonine, proline, hydroxyproline, β-alanine, N-butyl β-alanine, acide aminobutyrique, acide aminoca-proique, sarcosine, ou N-méthyl-β-alanine.

13. Composition selon l'une quelconque des revendications 10 à 12 où les huiles de formule (II) sont choisies parmi celles où p vaut 1 et la partie des esters aminoacides correspondant au groupe OR⁴ est obtenue à partir des alcools choisis dans le groupe formé par le méthanol, éthanol, propanol, isopropanol, butanol, tert-butanol, isobutanol, 3-méthyl-1-butanol, 2-méthyl-1-butanol, pentanol, héxanol, cyclohéxanol, octanol, 2-éthylhéxanol, décanol, alcool laurylique, alcool myristique, alcool cétylique, alcool cétostéarylique, alcool stéarylique, alcool oléique, alcool béhénylique, alcool de jojoba, alcool 2-héxadécylique, alcool 2-octyldodécanol et alcool isostéarylique.

14. Composition selon l'une quelconque des revendications 10 à 13 où l'huile de formule (II) est choisie parmi :
- le N-acétyl N-butylaminopropionate de formule suivante : 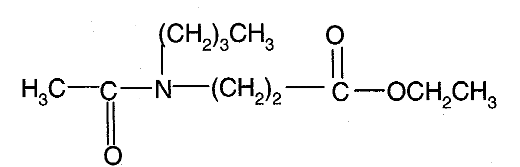
- le N-lauroylsarcosinate d'isopropyle de formule : 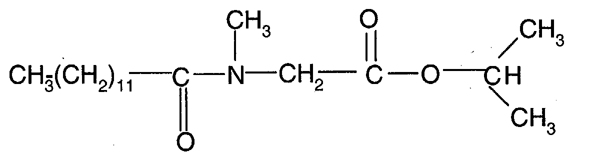
- le N,N-diéthyl-toluamide de formule :

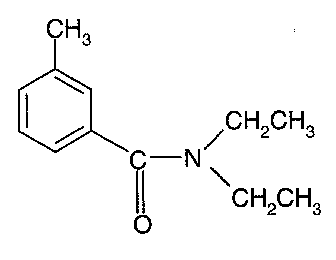

15. Composition selon l'une quelconque des revendications 1 à 14 où l'huile ou les huiles présentant dans leur structure au moins une fonction amide sont présentes à des concentrations allant de 0,1 à 40% en poids et plus préférentiellement de 1 à 20% en poids par rapport au poids total de la composition.

16. Composition selon l'une quelconque des revendications 1 à 15 où l'huile ou les huiles présentant dans leur structure au moins une fonction amide sont présentes dans une quantité suffisante permettant à elle(s) seule(s) de solubiliser dans le support la quantité totale de dérivé(s) de formule (I) dans la composition.

17. Composition selon l'une quelconque des revendications 1 à 16, contenant en plus au moins un agent filtrant les radiations UV.

18. Composition selon la revendication 17, où l'agent filtrant le rayonnement ultraviolet est choisi parmi les filtres UV organiques, les agents filtrant les radiations UV minéraux ou leurs mélanges.

19. Composition selon la revendication 17, où les filtres UV organiques sont choisis parmi les anthranilates ; les dérivés cinnamiques ; les dérivés de dibenzoylméthane ; les dérivés salicyliques, les dérivés du camphre ; les dérivés de triazine ; les dérivés de la benzophénone ; les dérivés de β,β'-diphénylacrylate ; les dérivés de benzotriazole ; les dérivés de benzimidazole ; les imidazolines ; les dérivés bis-benzoazolyle ; les dérivés de l'acide p-aminobenzoïque (PABA) ; les dérivés de méthylène bis-(hydroxyphényl benzotriazole) ; les polymères filtres et silicones filtres ; les dimères dérivés d'α-alkylstyrène ; les dérivés de 4,4-diarylbutadiène ; les hydroxybenzophénones aminées et leurs mélanges.

20. Composition selon la revendication 18, où les filtres UV organiques sont choisis parmi les composés suivants :
Ethylhexyl Salicylate,
Butyl Methoxydibenzoylmethane,
Ethylhexyl Methoxycinnamate,
Octocrylene,
Phenylbenzimidazole Sulfonic Acid,
Terephthalylidene Dicamphor Sulfonic Acid,.
Benzophenone-3,
Benzophenone-4,
Benzophenone-5,
2-(4-diethylamino-2-hydroxybenzoyl)-benzoate de n-hexyle
4-Methylbenzylidene camphor,
Disodium Phenyl Dibenzimidazole Tetra-sulfonate,
Anisotriazine,
Ethylhexyl triazone,
Diethylhexyl Butamido Triazone,
Methylene bis-Benzotriazolyl Tetramethylbutylphenol,
Drometrizole Trisiloxane,
Polysilicone-15
1,1-dicarboxy (2,2'-diméthyl-propyl)-4,4-diphénylbutadiène
et leurs mélanges.

21. Composition selon la revendication 18, où les agents filtrant minéraux sont des pigments ou bien encore des nanopigments d'oxydes métalliques enrobés ou non.

22. Composition selon la revendication 21 où les agents filtrant minéraux sont des nanopigments d'oxyde de titane, de fer, de zinc, de zirconium ou de cérium.

23. Composition selon l'une quelconque des revendications 18 à 22, où les agents filtrant les radiations sont présents dans des proportions allant de 0,1 à 20% en poids par rapport au poids total de la composition, et de préférence allant de 0,2 à 15% en poids par rapport au poids total de la composition.

24. Composition selon l'une quelconque des revendications 1 à 22, contenant en plus un ou plusieurs agents autobronzants.

25. Composition selon l'une quelconque des revendications 1 à 23, contenant en plus un ou plusieurs agents anti-pollution et/ou agent anti-radicalaire.

26. Composition selon l'une quelconque des revendications 1 à 24, contenant en plus un ou plusieurs inhibiteurs de lysyl et/ou prolyl hydroxylase.

27. Composition selon l'une quelconque des revendications 1 à 26, contenant en plus un ou plusieurs actifs choisis parmi les agents desquamants ; les agents hydratants ; les agents anti-inflammatoires ; les agents agissant sur le métabolisme énergétique des cellules ou leurs mélanges.

28. Composition selon l'une quelconque des revendications 1 à 27, contenant en plus un ou plusieurs actifs choisis parmi les agents dépigmentants et/ou les agents pro-pigmentants.

29. Composition selon l'une quelconque des revendications 1 à 28, contenant en outre un ou plusieurs adjuvants cosmétiques classiques choisis parmi les corps gras, les solvants organiques, les épaississants ioniques ou non ioniques, les adoucissants, les humectants, les opacifiants, les stabilisants, les émollients, les silicones, les agents anti-mousse, les agents répulsifs contre les insectes, les parfums, les conservateurs, les tensioactifs anioniques, cationiques, non-ioniques, zwitterioniques ou amphotères, les antagonistes de substance P, les antagonistes de substance CGRP, les charges, les polymères, les propulseurs, les agents alcalinisants ou acidifiants ou tout autre ingrédient habituellement utilisé dans le domaine cosmétique et/ou dermatologique.

30. Utilisation d'une composition telle que définie dans l'une quelconque des revendications précédentes pour la préparation d'un produit destiné à prévenir ou lutter contre les effets néfastes des radiations UV et/ou de la pollution sur les matières kératiniques humaines.

31. Utilisation d'une composition telle que définie dans l'une quelconque des revendications précédentes comme produit autobronzant et/ou pour le brunissage artificiel de la peau.

32. Utilisation d'une composition telle que définie dans l'une quelconque des revendications précédentes pour la préparation d'un produit destiné à prévenir la formation des radicaux libres au contact de la peau et/ou à neutraliser les radicaux libres déjà formés.

33. Utilisation d'une composition telle que définie dans l'une quelconque des revendications précédentes pour la préparation d'un produit destiné à traiter les cheveux endommagées par les effets néfastes des radiations UV et/ou de la pollution.

34. Utilisation d'une composition telle que définie dans l'une quelconque des revendications précédentes pour la préparation d'un produit destiné à traiter les peaux endommagées par les effets néfastes des radiations UV et/ou de la pollution

35. Utilisation d'une composition telle que définie dans l'une quelconque des revendications précédentes pour la préparation d'un produit destiné à traiter les peaux desséchées, craquelées, irritées ou brûlées par le soleil et/ou la pollution.

36. Utilisation d'une composition telle que définie dans l'une quelconque des revendications précédentes pour la préparation d'un produit destiné à prévenir et/ou à traiter les signes du vieillissement cutané, en particulier le photo-vieillissement de la peau

37. Utilisation d'une composition telle que définie dans l'une quelconque des revendications précédentes pour la préparation d'un produit destiné à prévenir et/ou traiter la perte de fermeté et/ou d'élasticité de la peau.

38. Utilisation cosmétique d'une composition telle que définie dans l'une quelconque des revendications précédentes pour prévenir et/ou réduire la formation de taches pigmentaires et/ou pour blanchir ou dépigmenter la peau.

39. Utilisation d'au moins une huile présentant dans sa structure au moins un motif amide telle que définie dans les revendications précédentes dans une composition cosmétique ou dermatologique comprenant dans un support contenant au moins une phase grasse au moins un dérivé ou un mélange de dérivés de la 1,7-bisphényl heptane-3,5-dione de formule (I) tel que défini dans les revendications précédentes pour solubiliser ledit dérivé ou mélange de dérivés de formule (I) dans le support.

40. Procédé de solubilisation d'au moins un dérivé ou un mélange de dérivés de la 1,7-bisphényl heptane-3,5-dione de formule (I) tels que définis dans les revendications précédentes dans une composition cosmétique ou dermatologique comportant au moins une phase grasse consistant à utiliser au moins une huile présentant dans sa structure au moins un motif amide telle que définie dans les revendications précédentes dans une quantité suffisante permettant de solubiliser à elle seule la totalité du ou des dérivés de la 1,7-bisphényl heptane-3,5-dione.
